# EUROPEAN PATENT APPLICATION

(11) **EP 3 660 145 A1**
(43) Date of publication of application: **03.06.2020**
(21) Application number: 18306590.3
(22) Date of filing: 30.11.2018
(51) Int. Cl.: C12N 5/079, A61K 35/30

(54) **USE OF OLIGODENDROCYTES FROM ORAL NEUROECTODERMAL STEM CELLS IN THE REPAIR OF THE NERVOUS SYSTEM**

(71) Applicant: ASSISTANCE PUBLIQUE, HOPITAUX DE PARIS, 75004 Paris (FR); Université de Paris 5 René Descartes, 75006 Paris (FR); Université Paris Diderot Paris 7, 75013 Paris (FR); Université Paris-Est Créteil Val de Marne, 94000 Créteil (FR)
(72) Inventor: FOURNIER, Benjamin, 75012 Paris (FR); GOGLY, Bruno, 77510 Hondevilliers (FR); MARCHAND-LEROUX, Catherine, 75016 Paris (FR); NASSIF, Ali, 78500 Sartrouville (FR); TAÏHI, Ihsène, 78500 Sartrouville (FR)
(74) Representative: Novagraaf Technologies

(57) **Abstract**

This invention concerns a new method for differentiating oral neuroectodermal stem cells (CSO-NE), in particular human gingival neuroectodermal stem cells (CSGh), into oligodendrocytes (OL), and their use in the repair of the nervous system, in particular of head injuries.

## Description

### Technical field of the invention

This invention concerns a new method for differentiating oral neuroectodermal stem cells (CSO-NE), in particular human gingival neuroectodermal stem cells (CSGh), into oligodendrocytes (OL), and their use in the repair of head injuries.

In the description below, references in square brackets ([ ]) refer to the list of references at the end of the text.

### State of the art

Brain injury or Head Injury/trauma (HI) remains, even today, one of the leading causes of mortality and morbidity in young adults. In Europe, the incidence of HI is about 235 per 100,000 hospitalized subjects per year (Tazaourte et al., 2008) [1], with an average mortality rate of about 15 per 100 000 hospitalized subjects. It is therefore a major public health problem because of its frequency, complexity, the severity of the sequelae involved and the high cost of health care. The main causes are road accidents, sports accidents, industrial accidents, domestic accidents and assaults. HI alters short-term consciousness and cognitive and physical abilities, leading to long-term behavioral or emotional problems. These disorders can be transient or permanent, leading to partial or total disability (Mathé et al., 2005) [2].

In recent years, in response to favourable results obtained in animals (Angoa Perez, 2014) [3], about fifteen clinical trials have evaluated the protective effects of neurons by several molecules (Narayan et al., 2002) [4]. Unfortunately, none of these studies have been able to demonstrate a therapeutic benefit in head injury patients (Bramlett and Dietrich, 2004; Beauchamp and Childress, 2008) [5, 6], leaving the medical profession largely helpless today in the face of trauma patients.

The severity of a HI is first of all related to the severity of the primary lesions that form at the time of the trauma, and then to the occurrence of secondary lesions in the hours, days, and months following the HI. Primary lesions consist of vascular, neural, glial or axonal lesions that occur at the time of the accident. These primary lesions trigger a neuro-inflammatory response that will play an important role in worsening the lesions and in the occurrence of long-term functional sequelae, including a risk of neurodegenerative and psychiatric disorders (Marmarou et al., 1994; Van Den Heuvel et al., 2007) [7, 8]. Post-traumatic injuries affect both gray and white matter. The latter are responsible for poor clinical prognosis with serious social consequences (Parvizi and Damasio, 2003) [9].

Among the white matter lesions, HI causes diffuse axonal lesions and demyelination of axons which comes from oligodendrocyte (OL) degeneration, cells responsible for myelin sheath formation (Biennow et al., 2012; Caprariello et al., 2012) [10, 11]. Oligodendrocytes play a crucial role since each is responsible for the myelination of an average of 50 neurons (Raine, 1984) [12]. These cells are cells that are vital to the functioning and survival of neurons. Without the protection by formation of the myelin sheath synthesized by the OLs, neurons quickly degenerate. The involvement or dysfunction of OLs therefore leads to serious pathologies of the central nervous system (CNS).

Myelin is the main component of the white matter. It facilitates the conduction of nerve impulses in the myelinated segments of axons. Myelin contains 80% lipids, some of which are specific to myelin such as galactoceramide, and 20% proteins such as myelin base protein or Myelin Basic Protein (MBP), oligodendrocyte myelin glycoprotein or Myelin Oligodendrocyte Glycoprotein (MOG), 2',3' -cyclic nucleotide phospho-diesterase (CNPase) (Hartline and Colman, 2007) [13].

CNS damage is known to activate several cell types that can influence the normal physiology of oligodendrocytes. Following a brain injury, macrophages from the blood are among the first cells to arrive at the site of the injury (Fawcett and Asher, 1999) [14]. These immune cells accumulate at the lesion site within a few hours of the injury. CNS microglia (OL) and macrophages phagocyte cell debris and foreign bodies and participate in inflammation, promotion and direction of tissue repair and maintenance of cell homeostasis (Den et al., 2015) [15).

Oral neuroectodermal stem cells isolated from oral mucous membranes, including the human gum (Human Gingival Stem Cells; CSGh), have recently been identified by the inventors (Fournier et al., 2010) [16]. These CSGs have the same embryological origin as maxillary bones, i.e. neural ridges. While many currently known adult stem cells derive from the endoderm, mesoderm or ectoderm and have specific markers that allow their isolation, the embryological origin of oral neuroectodermal stem cells gives them a particular plasticity, including their ability to differentiate into nerve cells, for example into oligodendrocytes. The latter are responsible for white matter repair and are currently considered key cells in brain injury repair.

There is therefore a need to open up new therapeutic prospects for head injuries for which the use of oligodendrocytes obtained from oral neuroectodermal stem cells in cell therapy could constitute a major scientific innovation. However, many essential developments are necessary, and suitable culture methods have yet to be defined before their use in cell therapy can be fully considered.

### Description of the invention

As part of the treatment of head injuries in humans or animals, the inventors have therefore developed a protocol for differentiating oral neuroectodermal stem cells (CSO-NE), in particular human gingival neuroectodermal stem cells (CSGh), into oligodendrocytes (OL).

Autologous CSO-NE or CSG were obtained without sequelae under local anesthesia (gingival sampling). The cells were easy to grow and proved to be particularly resistant. Gingival healing is considered embryo-like. Gingival tissue is probably the most aggressive tissue in the body (mechanical, thermal, chemical and bacterial aggressions), however no scars are visible in the oral cavity even after dental extractions.

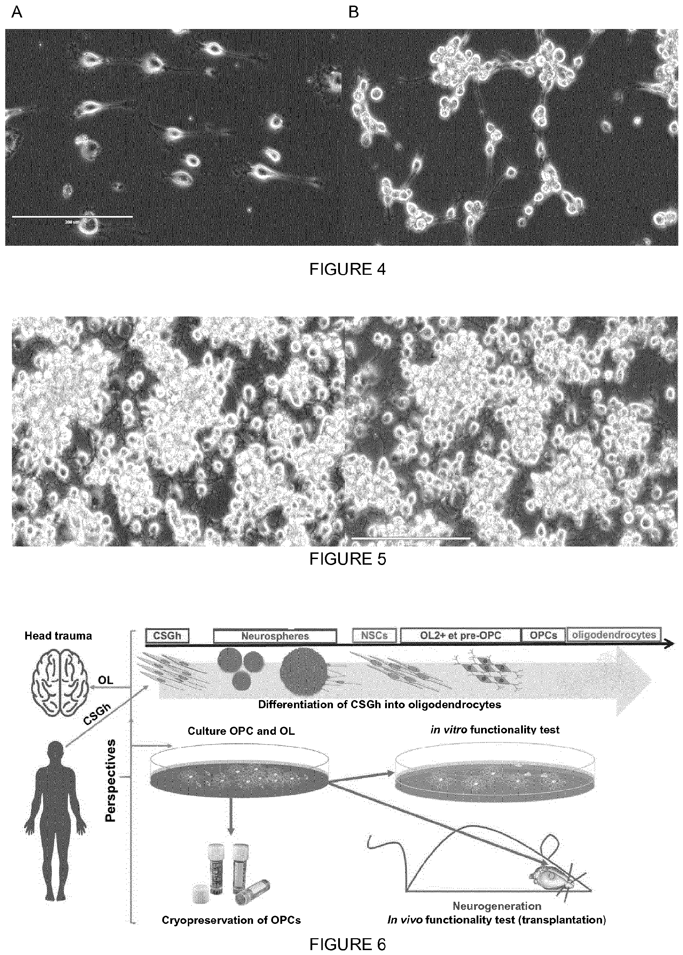

The inventors tested both the degree of maturation of oligodendrocytes obtained from CSGh and then improved the differentiation medium to obtain cells with a higher degree of maturity and better functional capacity. This functionality has been tested by demonstrating the ability of oligodencrocytes differentiated from CSGh to myelinate by co-cultivating them with neurons. Indeed, myelination by oligodendrocytes is a key element in the repair of central nervous tissue and the derivation of such cells from neuroectodermal gingival stem cells offers very innovative therapeutic prospects (Figure 6).

Inventors have thus established their protocol on the basis of several protocols that are described in the literature to differentiate embryonic stem cells and mesenchymal stem cells (Brüstle et al., 1999; Nistor et al., 2005; Chen et al., 2007; Hu et al., 2009; Najm et al., 2011; Leite et al., 2014) [17-22]. The protocol was then adapted according to the specific profile of CSO-NEs, and several tests performed on these cells to define the appropriate protocol for these cells.

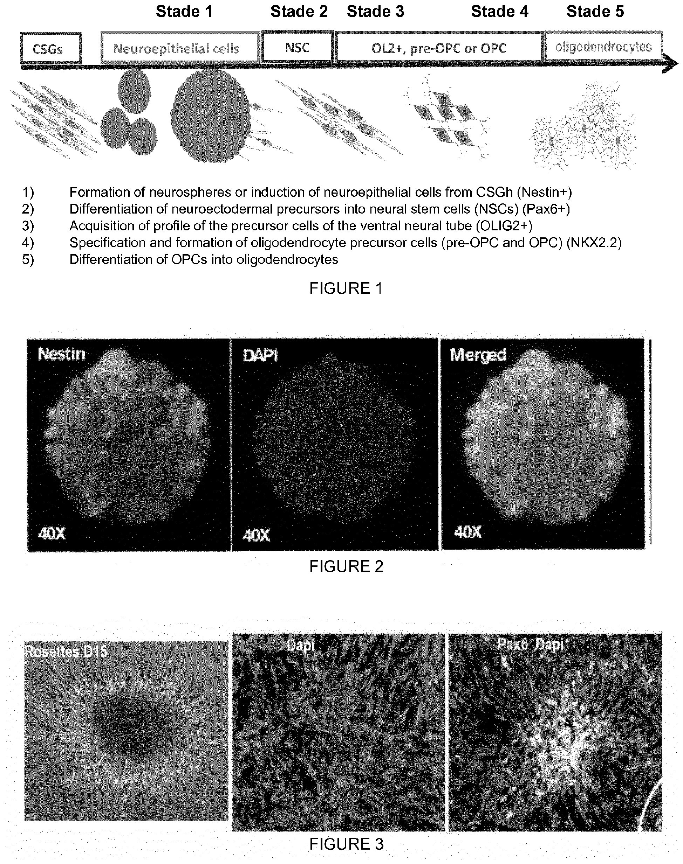

The inventors' protocol consists of several steps to change the profile of CSO-NE or CSGs into oligodendrocytes as shown in Figure 1.

Briefly, this protocol includes the induction of neural stem cells (NSCs) from CSO-NEs (or CSGs), the acquisition of the identity of neural progenitors that express OLIG2, the specification to early oligodendrocyte precursor cells (pre-OPCs) that express OLIG2/NKX2.2, and the generation of oligodendrocyte precursor cells (OPCs) and their differentiation into oligodendrocytes.

In the first part (Stades 1 and 2), the CSO-NE (or CSGs) are directed towards the fate of the neuroectoderm under a chemically defined condition for 2 weeks. Neuroepithelial differentiation can be reliably identified by observing the organization of these colonar epithelial cells that form rosettes, a characteristic topography of the neural tube from which OPCs derive during development (Marquardt and Pfaff, 2001; Pankratz et al, 2007; Hu et al., 2009) [23, 24 and 20] and the expression of neuroectodermal transcription factors, including PAX6 and SOX1 (Li et al., 2005; Pankratz et al., 2007) [25, and 24].

The second part consists of modelling neural stem cells (NSCs) to the progenitors of the ventral neural tube by adding retinoic acid (RA) and SHH factor within 10 days. The identity of progenitors is defined by their OLIG2 expression (Hu et al., 2009) [20]. These OLIG2+ cells can then differentiate into spinal motor neurons. To avoid this differentiation and promote OPC generation, basic fibroblast growth factor (FGF2 or FGFb) is used in the third part of the protocol for 10 days (Hu et al., 2009) [20]. At day 35, the progenitors co-express OLIG2 and NKX2.2 and can no longer differentiate into motor neurons (Hu et al., 2009; Hu and Zhang, 2009] [20, 26].

Co-expression of OLIG2 and NKX2.2 generally indicates oligodencrocyte precursor (OPC) identity (Qi et al., 2001; Zhou et al., 2001; Fu et al., 2002; Vallstedt et al., 2005; Hu et al., 2009) [27-30, 20]. However, these cells in their early differentiation phase do not possess the morphological, biochemical and functional characteristics of OPC. This is why some authors call them pre-OPC cells (Hu et al., 2009) [20]. These pre-OPCs are grown in a glial differentiation medium and more precisely an oligodendrocyte differentiation medium. This medium contains mainly triodothyronine (T3), Neurotrophin 3 (NT3), PDGF-AA, cAMP, IGF-1 and biotin, factors which, individually or synergistically, can promote the survival and proliferation of CSGh-derived OPCs. These OPCs have a bipolar or multipolar form and express OLIG2, NKX2.2 and PDGFRα Finally, these cells are mobile and can differentiate into oligodendrocyte-like cells.

An object of the present invention is therefore a method for inducing differentiation of oral neuroectodermal stem cells into oligodendrocytes, said method comprising or consisting of:
a) a step of differentiating said neuroectodermal oral stem cells into neuroepithelial stem cells in a neurogenic culture medium comprising DMEMf12, N1 supplement, FGFb and EGF.
b) a step of differentiating the neuroepithelial stem cells obtained in step a) into neural stem cells in a neurogenic culture medium comprising DMEMf12, N1 supplement, Noggin protein, FGFb, retinoic acid and SHH factor.
c) a step of differentiating the neural stem cells obtained in step b) into oligodendrocyte pre-precursor cells in a neurogenic culture medium comprising DMEMf12, N1 supplement, B27 supplement and FGFb.
d) a step of differentiating the oligodendrocyte pre-precursor cells obtained in step c) into oligodendrocyte precursor cells in a neurogenic culture medium comprising DMEMf12, PDGF-AA and SHH factor.
e) a step of differentiating oligodendrocyte precursor cells obtained in step d) into oligodendrocytes, and maturing said oligodendrocytes, in a neurogenic culture medium comprising DMEMf12, IGF-1, biotin, CAMP, PDGF-AA, L-glutamine or Glutamax™, and NT3.

According to a particular embodiment of the method of the present invention, neuroectodemic oral stem cells are derived from gingival tissue.

According to a particular embodiment of the method of the present invention, stade 1 is carried out over a period of 5 days, stade 2 is carried out over a period of 10 days, stade 3 in continuity with stade 4 are carried out over a period of 20 days, and/or stade 5 is carried out over a period of at least 15 days.

According to a particular embodiment of the method of the present invention, supplement N1 can be used at a concentration of about 0,1-1%; FGFb can be used at a concentration of about 10-100 ng/ml, for example at a concentration of about 50 ng/ml; EGF can be used at a concentration ranging from about 2-20 ng/ml, for example at a concentration of about 10 ng/ml; Noggin can be used at a concentration ranging from about 20-200 ng/ml, for example at a concentration of about 100 ng/ml; RA can be used at a concentration ranging from about 2-40 nM, for example at a concentration of about 20 nM; SHH can be used at a concentration ranging from about 20-400 ng/ml, for example at a concentration of about 100 or 200 ng/ml; PDGF-AA can be used at a concentration ranging from about 4-40 ng/ml, for example at a concentration of about 20 ng/ml.

Another object of the present invention is also a cell population comprising or consisting of oligodendrocytes obtained by the method of the present invention, said oligodendrocytes expressing the Msx1 gene (which is not the case for other OL obtained from other stem cells than stem cells from neural crests and/or other methods from prior art).

Another object of the present invention is also a cell population according to the present invention, for use in cellular therapy, preferably for the treatment of head injuries.

Another object of the present invention is also a culture medium for the differentiation of oral neuroectodermal stem cells into neuroepithelial stem cells comprising or essentially consisting of DMEMf12, N1 supplement, FGFb and EGF.

Another object of the present invention is also a culture medium for the differentiation of neuroepithelial stem cells into neural stem cells comprising or essentially consisting of DMEMf12, N1 supplement, Noggin protein, FGFb, retinoic acid and SHH factor.

Another object of the present invention is also a culture medium for the differentiation of neural stem cells into oligodendrocyte pre-precursor cells comprising or essentially consisting of DMEMf12, N1 supplement, B27 supplement and FGFb.

Another object of the present invention is also a culture medium for the differentiation of oligodendrocyte pre-precursor cells into oligodendrocyte precursor cells comprising or essentially consisting of DMEMf12, PDGF-AA and SHH factor.

Another object of the present invention is also a medium for culturing oligodendrocyte precursor cells into oligodendrocytes, and for maturing said oligodendrocytes, comprising or essentially consisting of DMEMf12, IGF-1, biotin, CAMP, PDGF-AA, L-glutamine or GlutamaxTM and NT3.

### Brief description of the figures

- Figure 1 shows the different steps of oligodendrocyte generation from CSGh.
- Figure 2 shows optical microscopy photos representing the neurospheres obtained from CSGh (stade 1 of the method of the present invention) and their characterization using different staining methods well-known to the skilled person. The neuroepithelial character was confirmed by the positive Nestin staining (specific for neurons). Cellular integrity was verified by nuclear staining with DAPI. The photo "merged" indicated the organization into neurospheres with a lot of Nestin staining at the periphery and a slightly less differentiated reserve of cells in the center. The size of the neurospheres can be measured. (40x magnification)
- Figure 3 shows optical microscopy photos representing the neural stem cells obtained after differentiation of the neuroepithelial cells obtained in stade 1 (stade 2 of the method of the present invention) and their characterization using different staining methods well-known to the skilled person. The organization into rosette (Day 15, no staining) was specific to neural stem cells cultures. Cellular integrity was verified by nuclear staining with DAPI (+ Nestin staining). Neural stem cells were further characterized by a positive Pax6 staining with DAPI.
- Figure 4 shows optical microscopy photos (no staining) representing (A) the ventral neural tube precursor cells obtained after differentiation of the neural stem cells obtained in stade 2 (stade 3 of the method of the present invention). The precursor cells of the ventral neural tube were characterized by positive OLIG2 staining (B) oligodendrocyte precursor cells (pre-OPC and OPC) differentiated from ventral neural tube precursor cells obtained in stade 3 (stade 4 of the method of the present invention). OL precursors (pre-OPC and OPC) were characterized by a positive NKX2.2 staining.
- Figure 5 shows optical microscopy photos (no staining) representing the mature oligodendrocytes obtained after differentiation of the oligodendrocyte precursors obtained at stade 4 (stade 5 of the method of the present invention). Mature oligodendrocytes have a characteristic cell morphology (small, branched central cell body.
- Figure 6 represents the principles and objectives of the invention: method of differentiation of CSG into oligodendrocytes, test of oligodendrocyte functionality and their use in cellular therapy for the repair of head injuries.

### EXAMPLES

### EXAMPLE 1 : DIFFERENTIATION PROTOCOL OF CSGh INTO OLIGODENDROCYTES

The different experiences were based on the differentiation of: PPSCs (pluripotent stem cells) (Hu et al., 2009 [20]), hESCs : (Human embryonic stem cells) (Nistor et al., 2005 [18]), and MSCs (mesenchimatous stem cells) (Leite et al., 2014 [22]).

| **Stade 1** | | | | | | | | |
|---|---|---|---|---|---|---|---|---|
| **Differentiation into neuroepithelial cells (CNE): neurospheres (****Figure 2****)** | | | | | | | | |
| Protocol | Cell line | Culture medium | Supplements | Type of culture | Treatment of culture dish | Culture dish | Cell dissociation | Culture time |
| **CSO-NE** | **CSO-NE** | **MN** | **N1, FGFb(50ng/ml), EGF(10ng/ml)** | **Suspended** | **Poly-Heme** | **65mm** | **Accutase** | **5 days** |
| Observations : Suspended culture in a neurogenic medium composed of: DMEMf12, N1 supplement, 0.5%, non-essential amino acids, 0.5%, antibiotic 0.5%, FGFb 50ng/ml, EGF 10ng/ml. | | | | | | | | |
| Remarks: In this part of suspended cell culture: 4 cell populations may appear: floating spheres, cell populations that adhere to the bottom of the dish despite treatment, adherent spheres and a last population of cells that have lipid vacuoles related to the presence of insulin in the N1 supplement (0.5mg/ml) (generally these are cells very sensitive to serum). For the passage to the next stade, only floating spheres containing cells capable of going beyond this first emancipation to the neural line are recovered. It should be noted that these spheres exhibit different sizes and densities and that dense spheres give rise to small spheres (5-10 cells) that proliferate over time. The culture time has been adjusted to 5 days to avoid any cell necrosis in the central part of the spheres and which seems sufficient to move to the next stade. These spheres are positive in Nestin, PIIITub (partially). | | | | | | | | |

| **Stade 2** | | | | | | | | |
|---|---|---|---|---|---|---|---|---|
| **Differentiation into neural stem cells (NSC): neuroepithelial stem cells (****Figure 3****)** | | | | | | | | |
| Protocol | Cell line | Culture medium | Supplements | Type of culture | Treatment of culture dish | Culture dish | Cell dissociation | Culture time |
| **CSO-NE** | **CSO-NE** | **MN** | **N1, Noggin(100ng/ml), FGFb(50ng/ml), RA(20nM), SHH(100ng/ml)** | **Adherent** | **untreated** | **65mm** | **Yes, Accutase** | **10 days** |
| Observations : Adherent cultures in a neurogenic environment composed of: DMEMf12, supplement N1. The cells that are well dissociated at first, begin to organize radially by forming what are called rosettes (simulating the organization of neural tube cells). The cells at this stade are in proliferation and still keep the fusiform shape. After 5 days, the rosettes become mature rosettes and they begin to connect with other rosettes until the confluence of the cells. | | | | | | | | |
| Remarks : Consideration was given to adding RA to neuroepithelial cells (mature rosette stade in the differentiation protocol) that carry the identity of cells derived from the dorsal anterior part of the neural tube and to differentiate them into OPCs that are in themselves derived from the ventral part of the tube; caudalizing factors such as the SHH factor or the RA that induces the expression of the Hox PG1 genes, the Nlz-1 and -2 genes, and vHnf1; these genes are essential for the development of this part and the development of OPC *in vivo.* | | | | | | | | |

| **Stade 3** | | | | | | | | |
|---|---|---|---|---|---|---|---|---|
| **Differentiation of oligodendrocytes into pre-precursor cells (Pre-OPC) (****Figure 4A****)** | | | | | | | | |
| Protocol | Cell line | Culture medium | Supplements | Type of culture | Treatment of culture dish | Culture dish | Cell dissociation | Culture time |
| **CSO-NE** | **CSO-NE** | **MN** | **N1, B27, FGFb (3 days)** | **Adherent** | **Laminin** | **65mm** | **In continuity with stade 4** | |
| Observations : The cells were sensitive to spherical culture: a very significant loss of cells between stade 2 and 4. The cells were kept adherent between these two stades with the changes of medium in order to direct them towards OPC, knowing that from this stade onwards several cells present a bipolar or multipolar shape with small branches reminiscent of the shape of oligodendrocyte progenitors. | | | | | | | | |
| Remarks : Cells tend to form spheres after the 10th day of culture, which explains why some authors use the suspended technique for this stade of differentiation. | | | | | | | | |

| **Stade 4** | | | | | | | | |
|---|---|---|---|---|---|---|---|---|
| **Differentiation into oligodendrocyte precursor cells (OPC) (****Figure 4B****)** | | | | | | | | |
| Protocol | Cell line | Culture medium | Supplements | Type of culture | Treatment of culture dish | Culture dish | Cell dissociation | Culture time |
| **CSO-NE** | **CSO-NE** | **MN** | **20ng/ml, PDGF-AA, 200ng/ml SHH** | **Adherent or suspended** | **Polyornithine, laminin** | **12-well plate** | **Accutase** | **20 days** |
| Observations : OPCs are in the form of small cells with bipolarity and several terminal branches, some of these cells have lipid vacuoles. These OPCs are better cultivated in suspension which promotes the differentiation of these cells and their maturation. FGFb and EGF were removed because these factors block cell differentiation. | | | | | | | | |
| Remarks : OPCs send extensions to connect with other OPCs and tend to cluster in 3-dimensional clusters and some reproduce the organization of the cells of the ventral neural tube. | | | | | | | | |

| **Stade 5** | | | | | | | | |
|---|---|---|---|---|---|---|---|---|
| **Differentiation of OPCs into oligodendrocytes and their maturation (OL) (****Figure 5****)** | | | | | | | | |
| Protocol | Cell line | Culture medium | Supplements | Type of culture | Treatment of culture dish | Culture dish | Cell dissociation | Culture time |
| **CSO-NE** | **CSO-NE** | **MN** | **IGF-1, T3, biotin, cAMP, PDGFAA, I-glutamine 2mM Glutamax™, NT3** | **Adherent** | **PDL, polyornithine and laminin** | **12-well plate** | **Accutase** | **>15 days** |
| Observations : Cells with a small central cell body and several branches | | | | | | | | |
| Remarks : The cells at this stade are cultured in islets: 10000 cells per 100µl of medium. | | | | | | | | |

| | | | | | | | | |
|---|---|---|---|---|---|---|---|---|
| PPSCs : pluripotent stem cells hESCs : Human embryonic stem cells MSCs : Mesenchymal stem cells CSO-NE : Oral neuroectodermal stem cells DMEMf12 : DMEM F12 Media \| Dulbecco Modified Eagle Medium, Gibco® MN : Neurogenic medium N1 : Supplement N1 (used to produce a complete growth medium for N1 neuronal cells or a closely related medium) EGF : epidermal growth factor FGFb (or FGF2) : basic fibroblast growth factor B27 : Supplement allowing the proliferation of cells, e.g. neuronal cells, without differentiation. IGF or IGF-1 : insulin-like growth factor 1 PDL : poly-D-lysine T3 : T3 factor (thyroid hormone T3) inducer for oligodendrocyte line precursors RA : retinoic acid SHH : protein factor Sonic HedgeHog (role in regulating the fate of neural stem cells) Noggin : (NOG) protein involved in the development of many tissues, including nervous tissues PDGF : Platelet-derived growth factor (regulates cell growth and division) PDGF-AA : AA isoform of platelet-derived growth factor (regulates cell growth and division) | | | | | | | | |

### List of references

1. Tazarourte et al. "(1), Bernard Vigué (2) Pôle Urgence SAMU 77.(1) Hôpital Marc Jacquet 77000 Melun.(2) DAR CHU de Bicêtre 94275 Le Kremlin Bicêtre Cedex. « Epidémiologie des traumatismes craniens », 2008
2. Mathé et al., Santé publique et traumatismes crâniens graves. Aspects épidémiologiques et financiers, structures et filières de soins. Annales francaises d'anesthesie et de reanimation, Elsevier.2005
3. Angoa Perez, Journal of neurochemistry 129(6): 916-931, 2014
4. Narayan et al., Journal of neurotrauma 19(5): 503-557, 2002
5. Bramlett et Dietrich, Journal of Cerebral Blood Flow & Metabolism 24(2): 133-150, 2004
6. Beauchamp et Childress, Les principes de l'éthique biomédicale, Les Belles Lettres.2008
7. Marmarou et al., Journal of neurosurgery 80(2): 291-300, 1994
8. Van Den Heuvel et al., Progress in brain research 161: 303-316, 2007
9. Parvizi et Damasio, Brain 126(7): 1524-1536, 2003
10. Biennow et al., Neuron 76(5): 886-899, 2012
11. Caprariello et al., Annals of neurology 72(3): 395-405, 2012
12. Raine, Laboratory Investigation 50(6): 608-635, 1984
13. Hartline et Colman, Current Biology 17(1): R29-R35, 2007
14. Fawcett et Asher, Brain Res Bull 49(6): 377-391, 1999
15. Dent et al., PLoS One 10(3): e0121541, 2015
16. Fournier et al., Tissue Engineering Part A, 16(9) : 2891-2899, 2010
17. Brüstle et al., Science, 285(5428) : 754-756, 1999
18. Nistor et al., Glia, 49(3) : 385-396, 2005
19. Chen et al., Nature Protocols, 2(5) : 1044-1051, 2007
20. Hu et al., Development, 136(9) ; 1443-1452, 2009
21. Hu, et al., Nature protocols 4(11): 1614-1622, 2009
22. Najm et al., Nature Methods, 8(11) : 957-962, 2011
23. Leite et al., PloS one, 9(10) : e111059, 2014
24. Marquardt et Pfaff, Cell, 106(6) : 651-654, 2001
25. Pankratz et al., Stem cells, 25(6) : 1511-1520, 2007
26. Li et al., Nature Biotechnology, 23(2) : 215-221, 2005
27. Hu et Zhang, Nature Protocols, 4(9) : 1295-1304, 2009
28. Qi et al., Development, 128(14) : 2723-2733, 2001
29. Zhou et al., Neuron, 31(5) : 791-807, 2001
30. Fu et al., Development, 129(3) : 681-693, 20022002
31. Vallstedt et al., Neuron, 45(1) : 55-67, 2005

## Claims

1. A method for inducing differentiation of oral neuroectodermal stem cells into oligodendrocytes, said method comprising:
a) a step of differentiating said neuroectodermal oral stem cells into neuroepithelial stem cells in a neurogenic culture medium comprising DMEMf12, N1 supplement, FGFb and EGF.
b) a step of differentiating the neuroepithelial stem cells obtained in step a) into neural stem cells in a neurogenic culture medium comprising DMEMf12, N1 supplement, Noggin protein, FGFb, retinoic acid and SHH factor.
c) a step of differentiating the neural stem cells obtained in step b) into oligodendrocyte pre-precursor cells in a neurogenic culture medium comprising DMEMf12, N1 supplement, B27 supplement and FGFb.
d) a step of differentiating the oligodendrocyte pre-precursor cells obtained in step c) into oligodendrocyte precursor cells in a neurogenic culture medium comprising DMEMf12, PDGF-AA and SHH factor.
e) a step of differentiating oligodendrocyte precursor cells obtained in step d) into oligodendrocytes, and maturing said oligodendrocytes, in a neurogenic culture medium comprising DMEMf12, IGF-1, biotin, CAMP, PDGF-AA, L-glutamine or GlutamaxTM and NT3.

2. A method according to claim 1, wherein the neuroectodemic oral stem cells are derived from gingival tissue.

3. A cell population comprising oligodendrocytes obtained by the method according to claim 1 or 2, said oligodendrocytes expressing the Msx1 gene.

4. Cell population as defined in claim 3, for use in cell therapy.

5. Cell population for use according to claim 4, for the treatment of head injuries.

6. Culture medium for the differentiation of oral neuroectodermal stem cells into neuroepithelial stem cells comprising DMEMf12, N1 supplement, FGFb and EGF.

7. Culture medium for the differentiation of neuroepithelial stem cells into neural stem cells comprising DMEMf12, N1 supplement, Noggin protein, FGFb, retinoic acid and SHH factor.

8. Culture medium for the differentiation of neural stem cells into oligodendrocyte pre-precursor cells comprising DMEMf12, N1 supplement, B27 supplement and FGFb.

9. Culture medium for the differentiation of oligodendrocyte pre-precursor cells into oligodendrocyte precursor cells comprising DMEMf12, PDGF-AA and SHH factor.

10. Culture medium for the differentiation of oligodendrocyte precursor cells into oligodendrocytes, and for the maturation of said oligodendrocytes, comprising DMEMf12, IGF-1, biotin, CAMP, PDGF-AA, L-glutamine or GlutamaxTM and NT3.
